# EUROPÄISCHE PATENTANMELDUNG

(11) **EP 0 784 049 A1**
(43) Veröffentlichungstag der Anmeldung: **16.07.1997**
(21) Anmeldenummer: 97100033.6
(22) Anmeldetag: 03.01.1997
(51) Int. Cl.: C07C 209/36, C07C 209/38

(54) **Verfahren zur Herstellung von gegebenenfalls substituierten 4-Aminodiphenylaminen**

(30) Priorität: 11.01.1996 DE 19600722
(71) Anmelder: BAYER AG, 51368 Leverkusen (DE)
(72) Erfinder: Reinartz, Klaus, Dr., 50765 Köln (DE); Brill, Adolf, 25548 Kellinghusen (DE); Schuhmacher, Fred, 25560 Schenefeld (DE)

(57) **Zusammenfassung**

4-Aminodiphenylamine werden hergestellt, indem man gegebenenfalls substituiertes Anilin mit gegebenenfalls substituiertem Nitrobenzol in Gegenwart von Wasser und/oder Alkoholen und organischen und/oder anorganischen Basen umsetzt und anschließend das erhaltene Nitro- und/oder Nitrosodiphenylamin katalytisch in Gegenwart von Wasser hydriert, wobei man die katalytische Hydrierung des Reaktionsgemischs in Gegenwart von 25 bis 80 Gew.-% Wasser, bezogen auf das Gewicht des Reaktionsgemisches aus der Kondensationsreaktion, durchführt, nach Beendigung der Wasserstoffaufnahme das Hydriergemisch vom Hydrierkatalysator befreit, gegebenenfalls dem Hydriergemisch 10 bis 100 Vol.-% aromatisches Lösungsmittel, bezogen auf das Gesamtvolumen des Hydriergemischs, zugibt, die gebildete organische Phase zur Isolierung des 4-Aminodiphenylamins abtrennt und die wäßrige Phase im Ausgangsreaktionsgemisch wieder zuführt.

## Beschreibung

Die Erfindung betrifft ein Verfahren zur Herstellung von gegebenenfalls substituierten 4-Aminodiphenylaminen (4-ADPA) durch Umsetzung von gegebenenfalls substituierten Anilinen mit gegebenenfalls substituierten Nitrobenzolen und anschließender katalytischer Hydrierung der erhaltenen Nitro- und/oder Nitrosodiphenylamine.

In der US-Patentschrift 5.117.063 wird ein Verfahren zur Herstellung von 4-Aminodiphenylaminen beschrieben, indem man gegebenenfalls substituierte Aniline mit gegebenenfalls substituiertem Nitrobenzol in Gegenwart von protischem Lösungsmittel und anorganischen und/oder organischen Basen umsetzt und die erhaltene Nitro- und/oder Nitrosodiphenylamine gegebenenfalls in Gegenwart von Wasser hydriert. Gemäß dem Beispiel 1 der genannten US-Patentschrift wird die katalytische Hydrierung in Gegenwart von 16 ml Wasser (ca. 10 Vol.-%) durchgeführt. Dabei soll die Ausbeute an 4-Aminodiphenylamin, bezogen auf eingesetztes Nitrobenzol, 85 % der Theorie betragen.

Neben der nur unbefriedigenden Ausbeute an 4-Aminodiphenylamin beinhaltet das in der genannten US-Patentschrift beschriebene Verfahren als weiteren Nachteil, daß die eingesetzte anorganische oder organische Base aufgrund des geringen Wasseranteils nur unvollständig zurückgewonnen werden kann. Dies geht zu Lasten der Wirtschaftlichkeit des dort beschriebenen Verfahrens. Da die eingesetzte Base nach dem Verfahren der US-Patentschrift 5.117.063 nur unvollständig zurückgewonnen werden kann, verbleibt die restliche Base in dem Rohgemisch, was bei der Aufarbeitung des Rohgemisches zu unerwünschten Neben- bzw. Zersetzungsprodukten führen kann.

Aufgabe der vorliegenden Erfindung war es nun, die oben beschriebenen Nachteile des in der US-Patentschrift 5.117.063 beschriebenen Verfahrens zu vermeiden und ein Verfahren zur Verfügung zu stellen, bei dem der eingesetzte basische Katalysator praktisch vollständig in das eingesetzte Reaktionsgemisch zurückgeführt werden kann. Darüber hinaus soll das erfindungsgemäße Verfahren zu hohen Ausbeuten an 4-Aminodiphenylaminen führen und praktisch kaum Neben- bzw. Zersetzungsprodukte bei der Aufarbeitung liefern.

Gegenstand der vorliegenden Erfindung ist daher ein Verfahren zur Herstellung von gegebenenfalls substituiertem 4-Aminodiphenylamin durch Umsetzung von gegebenenfalls substituiertem Anilin mit gegebenenfalls substituiertem Nitrobenzol in Gegenwart von Wasser und/oder Alkoholen und anorganischen und/oder organischen Basen und anschließender katalytischer Hydrierung des erhaltenen Nitro- und/oder Nitrosodiphenylamins in Gegenwart von Wasser, das dadurch gekennzeichnet ist, daß man die katalytische Hydrierung in Gegenwart von 25 bis 80 Gew.-% Wasser, bezogen auf das Gewicht des Reaktionsgemisches aus der Kondensationsreaktion, durchführt, nach Beendigung der Wasserstoffaufnahme das Hydriergemisch vom Hydrierkatalysator befreit, die gebildete organische Phase zur Isolierung des 4-Aminodiphenylamins abtrennt und die wäßrige Phase dem Ausgangsreaktionsgemisch wieder zuführt.

Bevorzugt wird die Hydrierung nach dem erfindungsgemäßen Verfahren in Gegenwart von 30 bis 50 Gew.-% Wasser, bezogen auf das Gesamtgewicht des Reaktionsgemisches, durchgeführt.

Nachdem das Hydriergemisch von dem Hydrierkatalysator befreit wurde, wird dem Hydriergemisch bevorzugt 10 bis 100 Vol.-%, insbesondere 10 bis 80 Vol.-%, bezogen auf das Gesamtvolumen des Hydriergemisches, aromatisches Lösungsmittel zudosiert. Als aromatische Lösungsmittel seien genannt: Benzol, Toluol und/oder Xylol, bevorzugt Toluol.

Als gegebenenfalls substituierte Aniline können in das erfindungsgemäße Verfahren eingesetzt werden: Anilin; o-, m- oder p-Methylanilin; o-, m- oder p-Ethylanilin; o-, m- oder p-Methoxyanilin, bevorzugt Anilin.

Als gegebenenfalls substituierte Nitrobenzole können in das erfindungsgemäße Verfahren eingesetzt werden: Nitrobenzol; o- oder m-Methylnitrobenzol; o- oder m-Ethylnitrobenzol; o- oder m-Methoxynitrobenzol, insbesondere Nitrobenzol.

Üblicherweise werden bei dem erfindungsgemäßen Verfahren pro mol Nitrobenzol 1 bis 10, insbesondere 3 bis 6 mol Anilin eingesetzt.

Für das erfindungsgemäße Verfahren ist es wichtig, daß in Gegenwart von protischen Lösungsmitteln wie Wasser und/oder Alkoholen, wie Methanol oder Ethanol, gearbeitet wird. Dabei sollte die Menge an protischem Lösungsmittel 8 Vol.-%, bezogen auf das Gesamtvolumen des Reaktionsgemisches, nicht übersteigen. Vorteilhafterweise sollte die Menge an eingesetztem protischem Lösungsmittel nicht mehr als 4 Vol.-% betragen. Die eingesetzte Menge an protischen Lösungsmitteln hängt dabei von zusätzlich eingesetzten Lösungsmitteln, wie Dimethylsulfoxid, Dimethylformamid, Pyridin, Toluol und/oder Hexan, ab und ist leicht durch Vorversuche zu ermitteln.

Bevorzugt wird das erfindungsgemäße Verfahren in Gegenwart von Wasser und/oder Methanol durchgeführt, insbesondere in Gegenwart von Wasser. Das optimale Mischungsverhältnis kann durch entsprechende Vorversuche ermittelt werden.

Als anorganische und/oder organische Basen sind für das erfindungsgemäße Verfahren insbesondere geeignet: Alkalimetalle, Alkalimetallhydroxide, tetrasubstituierte Ammoniumhydroxide, gegebenenfalls auch in Gegenwart von Phasentransferkatalysatoren. Ganz besonders bevorzugt sind Tetraalkylammoniumhydroxide, insbesondere Tetramethylammoniumhydroxid.

Die Basen werden üblicherweise in Mengen von 1 bis 4 mol, bevorzugt 1 bis 1,5 mol, bezogen auf 1 mol Nitrobenzol, eingesetzt, wobei das Molverhältnis von protischem Lösungsmittel zu Base üblicherweise 1:1 bis 4:1 beträgt.

Die Umsetzung des gegebenenfalls substituierten Anilins mit dem gegebenenfalls substituierten Nitrobenzol wird im allgemeinen bei Temperaturen von 50 bis 100°C, bevorzugt 60 bis 80°C, durchgeführt. Man arbeitet dabei mit Unterdruck, d.h. bei Drücken von 20 bis 150 mbar, bevorzugt von 70 bis 80 mbar.

Nach dem erfindungsgemäßen Verfahren ist es möglich, das bei der Umsetzung der genannten Aniline mit den genannten Nitrobenzolen erhaltene Reaktionsgemisch direkt einer katalytischen Hydrierung zu unterwerfen. Selbstverständlich ist es auch möglich, die erhaltenen Zwischenprodukte, d.h. Nitro- und/oder Nitrosodiphenylamine, in geeigneter Weise zu isolieren und dann einer katalytischen Hydrierung zu unterwerfen. Bei der Isolierung der Nitro- und/oder Nitrosodiphenylamine, die in Form der Salze zunächst erhalten werden, ist es möglich, die Salze in die freien Verbindungen durch Hydrolyse überzuführen und die freien Verbindungen dann der katalytischen Hydrierung zu unterwerfen.

Bevorzugt wird jedoch das erfindungsgemäße Verfahren in der Weise durchgeführt, daß man die erhaltenen Nitro- und/oder Nitrosodiphenylamine direkt einer katalytischen Hydrierung in Gegenwart von Wasser und/oder Alkoholen unterwirft.

Die erfindungsgemäße Hydrierung kann mit den üblichen Hydrierungskatalysatoren, wie Edelmetalle auf Aktivkohle, Raney-Nickel, Raney-Kupfer, bevorzugt Edelmetalle auf Aktivkohle, insbesondere Platin auf Aktivkohle, durchgeführt werden. Solche Hydrierungskatalysatoren sind beispielsweise beschrieben in Ullmanns Encyklopädie der technischen Chemie, 4. Auflage, Band 13, S.141.

Die Menge an eingesetztem Hydrierungskatalysator beträgt üblicherweise 0,1 bis 1 Massen-%, bezogen auf die zu hydrierende Substanz.

Die katalytische Hydrierung wird bei Temperaturen von 50 bis 150°C, bevorzugt 60 bis 80°C durchgeführt. Der Wasserstoffdruck beträgt etwa 1 bis 25, insbesondere 10 bis 15 bar.

Nach Beendigung der Wasserstoffaufnahme wird das Hydriergemisch von dem Hydrierkatalysator befreit, beispielsweise durch Filtration. Anschließend wird dem Hydriergemisch gegebenenfalls die oben beschriebene Menge an aromatischem Lösungsmittel zugegeben, die dabei gebildete organische Phase zur Isolierung des 4-Aminodiphenylamins in üblicher Weise abgetrennt und die wäßrige Phase, die die organischen und/oder anorganischen Basen enthält, dem Ausgangsreaktionsgemisch wieder zugeführt.

Nach dem erfindungsgemäßen Verfahren wird 4-Aminodiphenylamin in einer Ausbeute von mindestens 86 % der Theorie, insbesondere mindestens 90 % der Theorie erhalten. Die eingesetzte organische und/oder anorganische Base wird zu mindestens 99 % zurückgewonnen. Dabei ist besonders hervorzuheben, daß die erfindungsgemäße Hydrierung mit einem gegenüber dem Stand der Technik erhöhtem Wassergehalt eine deutlich verkürzte Reaktionszeit und eine beträchtliche Erhöhung der Ausbeute an Hydrierungsprodukt ergibt.

### Beispiele

### Beispiel 1

18,7 kg 25 %iger wäßriger Tetramethylammoniumhydroxid-Lösung (TMAOH) werden bei einer Temperatur von 55°C und einem Druck von 75 mbar destillativ zu einer 35 %igen Lösung aufkonzentriert.

Nach Zugabe von 26,9 L Anilin wird bei einer Temperatur von 75°C und einem Druck von 75 mbar ein Anilin/Wasser-Azeotrop abdestilliert, bis das Molverhältnis Wasser:Base etwa 4:1 beträgt. Anschließend werden bei gleichen Bedingungen innerhalb von 3 Stunden 6,0 kg Nitrobenzol zugepumpt und das Gemisch 4 Stunden nachgerührt. Während dieser Zeit wird weiterhin ein Wasser/Anilin-Azeotrop abdestilliert.

Zu diesem Rohgemisch werden 220 g Pt/C-Katalysator (5 % Pt) und 12 L Wasser gegeben. Anschließend werden bei einer Temperatur von 80°C maximal 15 bar Wasserstoff aufgedrückt und das Reaktionsgemisch solange gerührt, bis keine Wasserstoffaufnahme mehr zu beobachten ist. Gemäß HPLC war die Ausbeute an Hydrierungsprodukten quantitativ. Die Reaktionszeit beträgt bei dieser Versuchsführung 4 Stunden. Es werden 10 L Toluol zugesetzt, der Katalysator abfiltriert und die organische und die wäßrige Phase in einem Scheidegefäß getrennt.

Die organische Phase wird anschließend durch fraktionierte Destillation aufgearbeitet. Die Reinausbeute an 4-ADPA beträgt 91 %, bezogen auf eingesetztes Nitrobenzol.

Die Analyse der wäßrigen Phase zeigt, daß 99,7 % des eingesetzten Tetramethylammoniumhydroxids isoliert werden können. Die erhaltene wäßrige Phase kann ohne Reaktivitätsverlust wieder in die Reaktion zurückgeführt werden.

### Beispiel 2

Anilin und Nitrobenzol werden analog Beispiel 1 in Gegenwart von Tetramethylammoniumhydioxid umgesetzt.

Zu diesem Rohgemisch werden Wassermengen (siehe Tabelle) hinzugefügt und der Pt/C-Katalysator zugegeben. Der Ansatz wird bei einer Temperatur von 80°C und einem Wasserstoffdruck von maximal 15 bar hydriert, bis keine Wasserstoffaufnahme mehr beobachtet werden kann.

Anschließend wird Toluol zugegeben und der Katalysator abgetrennt. Die wäßrige und die organische Phase werden getrennt. Die organische Phase wird gegebenenfalls mit frischem Wasser gewaschen, bis das Tetramethylammoniumhydroxid quantitativ extrahiert worden ist.

Die organische Phase wird dann fraktioniert destilliert. Die Reinausbeuten an 4-ADPA, bezogen auf eingesetztes Nitrobenzol, sind in folgender Tabelle zusammengestellt:

**Tabelle**

| Wassermenge bezogen auf eingesetztes Rohgemisch Gew.-% | Reinausbeute 4-ADPA bezogen auf eingesetztes Nitrobenzol % |
|---|---|
| 10* | 83 |
| 20* | 84 |
| 30 | 92 |
| 33 | 91 |
| 50 | 88 |
| 70 | 86 |
| 80 | 86 |
| 100* | 82 |

| | |
|---|---|
| * nicht erfindungsgemäß | |

## Patentansprüche

1. Verfahren zur Herstellung von gegebenenfalls substituiertem 4-Aminodiphenylamin durch Umsetzung von gegebenenfalls substituiertem Anilin mit gegebenenfalls substituiertem Nitrobenzol in Gegenwart von Wasser und/oder Alkoholen und organischen und/oder anorganischen Basen und anschließender katalytischer Hydrierung des erhaltenen Nitro- und/oder Nitrosodiphenylamins in Gegenwart von Wasser, dadurch gekennzeichnet, daß man die katalytische Hydrierung des Reaktionsgemischs in Gegenwart von 25 bis 80 Gew.-% Wasser, bezogen auf das Gewicht des Reaktionsgemisches aus der Kondensationsreaktion, durchführt, nach Beendigung der Wasserstoffaufnahme das Hydriergemisch vom Hydrierkatalysator befreit, gegebenenfalls die gebildete organische Phase zur Isolierung des 4-Aminodiphenylamins abtrennt und die wäßrige Phase dem Ausgangsreaktionsgemisch wieder zuführt.

2. Verfahren nach Anspruch 1, dadurch gekennzeichnet, daß man die katalytische Hydrierung in Gegenwart von 30 bis 50 Gew.-% Wasser durchführt.

3. Verfahren nach Ansprüchen 1 und 2, dadurch gekennzeichnet, daß man dem Hydriergemisch 10 bis 100 Vol.-% aromatisches Lösungsmittel zugibt.

4. Verfahren nach Ansprüchen 1 bis 3, dadurch gekennzeichnet, daß man die Umsetzung des Anilins mit Nitrobenzol bei Temperaturen von 50 bis 100°C durchführt.

5. Verfahren nach Ansprüchen 1 bis 4, dadurch gekennzeichnet, daß man pro Mol Nitrobenzol 1 bis 10 mol Anilin einsetzt.
